(19) Europäisches Patentamt
European Patent Office
Office européen des brevets

(11) **EP 3 789 767 B1**

(12) **EUROPEAN PATENT SPECIFICATION**

(45) Date of publication and mention
of the grant of the patent:
**08.04.2026 Bulletin 2026/15**

(51) International Patent Classification (IPC):
**G01N 33/543** (2006.01)     **G01N 21/65** (2006.01)
**G01N 33/569** (2006.01)

(21) Application number: **19795893.7**

(52) Cooperative Patent Classification (CPC):
**G01N 33/54346; G01N 21/658; G01N 33/56983;**
G01N 33/54353

(22) Date of filing: **02.05.2019**

(86) International application number:
**PCT/KR2019/005250**

(87) International publication number:
**WO 2019/212248 (07.11.2019 Gazette 2019/45)**

(54) **BACTERIOPHAGE-BASED SERS-ACTIVE GOLD NANOHALO STRUCTURE AND MANUFACTURING METHOD THEREFOR**

BAKTERIOPHAGENBASIERTE SERS-AKTIVE GOLD-NANOHALO-STRUKTUR UND HERSTELLUNGSVERFAHREN DAFÜR

STRUCTURE DE NANOHALO D'OR ACTIF EN DRES À BASE DE BACTÉRIOPHAGE ET SON PROCÉDÉ DE FABRICATION

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **02.05.2018 KR 20180050838**

(43) Date of publication of application:
**10.03.2021 Bulletin 2021/10**

(73) Proprietor: **Korea University Research and Business Foundation**
**Seoul 02841 (KR)**

(72) Inventors:
• **SIM, Sang Jun**
**Seoul 06276 (KR)**
• **JEON, Myoungjin**
**Gyeonggi-do 16708 (KR)**
• **MA, Xingyi**
**Seoul 02850 (KR)**

(74) Representative: **Mewburn Ellis LLP**
**Aurora Building**
**Counterslip**
**Bristol BS1 6BX (GB)**

(56) References cited:
**WO-A1-2011/130332     KR-A- 20170 131 864**

• **PARENT K N ET AL: "P22 Coat Protein Structures Reveal a Novel Mechanism for Capsid Maturation: Stability without Auxiliary Proteins or Chemical Crosslinks", STRUCTURE, ELSEVIER, AMSTERDAM, NL, vol. 18, no. 3, 10 March 2010 (2010-03-10), pages 390 - 401, XP026941383, ISSN: 0969-2126, [retrieved on 20100311], DOI: 10.1016/J.STR.2009.12.014**
• **EVERTS MAAIKE ET AL: "Covalently Linked Au Nanoparticles to a Viral Vector:? Potential for Combined Photothermal and Gene Cancer Therapy", NANO LETTERS, vol. 6, no. 4, 1 April 2006 (2006-04-01), US, pages 587 - 591, XP055885720, ISSN: 1530-6984, DOI: 10.1021/ nl0500555**
• **PUMPENS PAUL ET AL: "The True Story and Advantages of RNA Phage Capsids as Nanotools", INTERVIROLOGY., vol. 59, no. 2, 20 December 2016 (2016-12-20), CH, pages 74 - 110, XP055883746, ISSN: 0300-5526, DOI: 10.1159/ 000449503**

**(Cont. next page)**

- NIIKURA, K.: "Gold nanoparticle arrangement on viral particles through carbohydrate recognition: a non-cross-linking approach to optical virus detection", BIOCONJUGATE CHEMISTRY, 9 November 2009 (2009-11-09), XP055649803
- LEE, J: "Nanoscale bacteriophage biosensors beyond phage display", INTERNATIONAL JOURNAL OF NANOMEDICINE, vol. 8, 2013, XP055649808
- YUCE, M: "How to make nanobiosensors: Surface modification and characterisation of nanomaterials for biosensing applications", RSC ADVANCES, 2017, XP055649809
- DE LIU: "Enhanced plasmon resonance light scattering signals of colloidal gold resulted from its interactions with organic small molecules using captopril as an example", ANALYTICA CHIMICA ACTA, 29 June 2006 (2006-06-29), XP027912875
- PETRESCU, D. S.: "Viral-based nanomaterials for plasmonic and photonic materials and devices", WILEY INTERDISCIPLINARY REVIEWS: NANOMEDICINE AND NANOBIOTECHNOLOGY, vol. 10, e1508, 8 February 2018 (2018-02-08), pages 1 - 29, XP055649815

## Description

### Technical Field

The present invention relates to a bacteriophage-based gold nanohalo structure having surface-enhanced Raman scattering (SERS) activity and a method for fabricating the same, and more particularly to a bacteriophage MS2-based gold nanohalo structure in which multiple gold nanoparticles are uniformly bound to a bacteriophage via a linker at regular intervals, wherein the linker is (2S)-1-[(2S)-2-methyl-3-sulfanylpropanoyl]pyrrolidine-2-carboxylic acid (captopril).

### Background Art

Surface-enhanced Raman scattering (SERS) technology has recently attracted attention in the detection field due to its high sensitivity and specificity to a detection target. The oscillation of electrons occurring on the metal surface and the electrical interaction of analytes around the metal surface greatly amplify the characteristic Raman scattering spectrum of the analyte molecules. In a SERS-based sensor, the biggest factor in determining the sensitivity and stability of a signal is a metal substrate that functions to amplify a signal generated by an analyte, and localized surface plasmon resonance (LSPR) that occurs on the surface of a nanoscale metal structure enhances the strength and stability of the signal (A.H. Nguyen et al., 2015, Journal of Optics, 17, 114022).

An aggregate obtained by aggregating gold or silver nanoparticles using a salt or the like is frequently used as a substrate for SERS, and Raman signals can be greatly amplified by forming multiple hot spots between the nanoparticles (B. Sharma et al., 2013, MRS Bulletin, 38, 615-624). Nanoparticles having various shapes, such as nanospheres, nanorods, nanostars, and nanowires have been applied to this SERS substrate, and the substrate fabricated in this manner can amplify the Raman signal of analyte molecules by $10^9$ times or more. However, it is impossible to reproduce the structure of a SERS-active substrate based on this aggregate, and low signal reproducibility due to this has been continuously pointed out (H. Liu et al., 2011, Scientific Reports, 1, 112).

Meanwhile, the coat protein of bacteriophage MS2 has an icosahedral structure of about 28 nm in size, and is composed of 180 identical protein units. The MS2 coat protein is self-assembled into a regular and uniform structure by internal RNA, and a total of 360 modifiable amine functional groups are regularly arranged over the entire outer surface of the coat protein. The overall structure of bacteriophage MS2 is very stable physically and chemically, and thus bacteriophage MS2 has been used as delivery vehicle that delivers various substances such as peptides, polymers, and DNA aptamers, attached to the surface thereof through surface modification (S.L. Capehart et al., 2013, Journal of American Chemical Society, 135, 3011-3016).

Accordingly, the present inventors have made extensive efforts to develop a SERS substrate having high reproducibility, which overcomes the disadvantages of conventional SERS substrates. As a result, the present inventors have found that, when gold nanoparticles are uniformly bound to the surface of bacteriophage MS2 via a linker having a carboxyl group and a thiol group, a nanohalo structure having a uniform shape is fabricated and the magnitude of SERS signal amplification is also increased, thereby completing the present invention.

The above information disclosed in this Background section is only for enhancement of understanding of the background of the present invention. Therefore, it may not contain information that forms conventional art that is already known in the art to which the present invention pertains.

WO 2011/130332A1 relates to "glycan arrays for high throughput screening of viruses". PARENT K N ET AL (Structure. 2010 Mar 10; 18(3): 390-401.) relates "P22 Coat Protein Structures" which "Reveal a Novel Mechanism for Capsid Maturation". EVERTS MAAIKE ET AL (Nano Lett. 2006 Apr;6(4):587-91.) relates to "Covalently Linked Au Nanoparticles to a Viral Vector" and their "Potential for Combined Photothermal and Gene Cancer Therapy".

### Summary of the Invention

An object of the present invention is to provide a bacteriophage-based gold nanohalo structure.

Another object of the present invention is to provide a method for fabricating a bacteriophage-based gold nanohalo structure.

Still another object of the present invention is to provide the use of a bacteriophage-based gold nanohalo structure.

To achieve the above objects, the present invention provides a gold nanohalo structure in which gold nanoparticles are bound via a linker containing a carboxyl group and a thiol group to a bacteriophage having an amine functional group arranged on the surface thereof, wherein the linker is (2S)-1-[(2S)-2-methyl-3-sulfanylpropanoyl]pyrrolidine-2-carboxylic acid (captopril).

The present invention also provides a method for fabricating a gold nanohalo structure, the method comprising steps of: (a) activating a carboxyl group of a linker; (b) binding a thiol group of the linker to gold nanoparticles; and (c) reacting the carboxyl group of the linker bound to the gold nanoparticles with an amine group present on the surface of a bacteriophage, wherein the linker is (2S)-1-[(2S)-2-methyl-3-sulfanylpropanoyl]pyrrolidine-2-carboxylic acid (captopril) .

The present invention also provides a SERS composition comprising the gold nanohalo structure, for detection of a target substance.

**Brief Description of Drawings**

FIG. 1 schematically shows a process for fabricating a gold nanohalo structure according to the present invention.
FIG. 2(A) is a TEM image of negatively stained bacteriophage MS2, FIG. 2(B) is a TEM image of 5-nm gold nanoparticles, and FIG. 2(C) is a TEM image of a fabricated gold nanohalo structure.
FIG. 3(A) shows the size distributions of 5-nm gold nanoparticles, a gold nanohalo structure, and MS2 phage, analyzed by dynamic light scattering (DLS), and FIG. 3(B) is a graph showing changes in UV-Vis extinction spectra during fabrication of a gold nanohalo structure.
FIG. 4 is a graph showing changes in FT-IR spectra, which respectively occur when a captopril linker is bound to 5-nm gold nanoparticles and when MS2 phage is bound to the linker-bound gold nanoparticles to form a gold nanohalo structure.
FIG. 5 is a graph comparing the Raman spectrum of malachite green Raman dye with the enhanced Raman spectrum whose signal was amplified by a gold nanohalo structure.
FIG. 6(A) is a graph comparing the Raman spectra of malachite green, which were measured three times and whose signals were amplified by a gold nanohalo structure, and FIG. 6(B) is a graph showing the mean and standard deviation of the signal intensities of five main peaks in the spectra shown in FIG. 6(A).

**Detailed Description and Preferred Embodiments of the Invention**

Unless otherwise defined, all technical and scientific terms used in the present specification have the same meanings as commonly understood by those skilled in the art to which the present disclosure pertains. In general, the nomenclature used in the present specification is well known and commonly used in the art.

In the present invention, the SERS activity of a bacteriophage-based gold nanohalo structure was analyzed.

The present inventors fabricated gold nanoparticle complexes on the surfaces of various types of bacteriophages using a linker in order to fabricate gold nanoparticle complexes with regular intervals, but found that the fabrication of gold nanohalo structure was possible only on bacteriophage MS2.

This is because various types of bacteriophages contain capsid proteins with different structures, and only MS2 is the phage in which amine groups are exposed on the surface thereof at regular intervals.

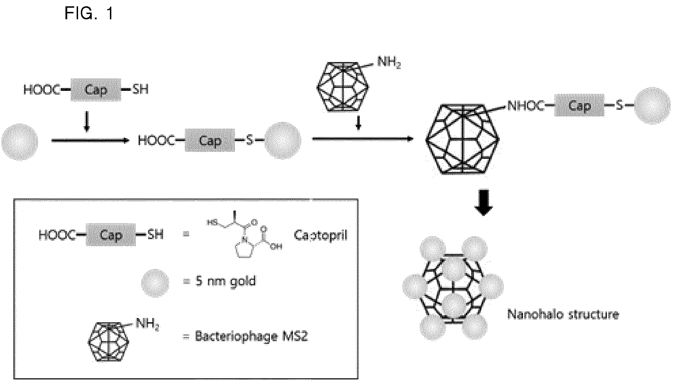

That is, in one example of the present invention, the carboxyl group of captopril containing the carboxyl group and a thiol group was activated by treatment with N-hydroxysuccinimide (NHS) and N-ethyl-N'-(3-dimethylaminopropyl) carbodiimide (EDC), and gold nanoparticles were bound to the thiol group of the captopril and bound to the amine group on the surface of bacteriophage MS2 via the carboxyl group of the captopril, thereby fabricating a bacteriophage-based gold nanohalo structure (FIG. 1).

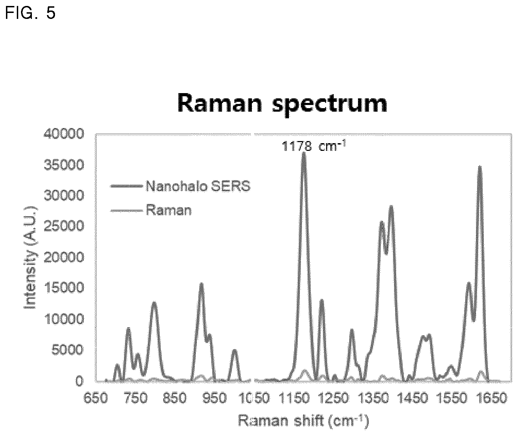

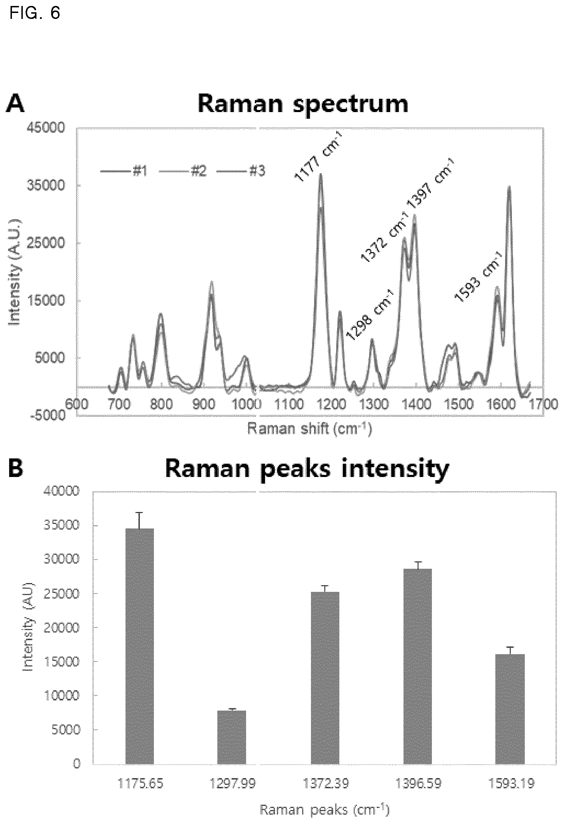

As a result, it was confirmed that the SERS signal amplification effect of the gold nanohalo structure was better than that of pristine gold nanoparticles (FIG. 5), and that the gold nanohalo structure exhibited excellent signal reproducibility in several measurements for one type of analyte (FIG. 6).

Therefore, in one aspect, the present invention is directed to a gold nanohalo structure comprising: (a) a bacteriophage having an amine functional group arranged on the surface thereof; (b) a linker containing a carboxyl group and a thiol group; and (c) gold nanoparticles, wherein the linker is (2S)-1-[(2S)-2-methyl-3-sulfanylpropanoyl]pyrrolidine-2-carboxylic acid (captopril).

In the present invention, the gold nanohalo structure may have surface-enhanced Raman scattering (SERS) activity.

In the present invention, the bacteriophage may be bacteriophage MS2, but is not limited thereto.

In the present invention, the linker is (2S)-1-[(2S)-2-methyl-3-sulfanylpropanoyl]pyrrolidine-2-carboxylic acid (captopril).

In the present invention, the size of the gold nanoparticles may be 1 to 100 nm, but is not limited thereto.

In another aspect, the present invention is directed to a method for fabricating a gold nanohalo structure, the method comprising steps of: (a) activating a carboxyl group of a linker containing the carboxyl group and a thiol group; (b) binding a thiol group of the linker to gold nanoparticles; and (c) reacting the carboxyl group of the linker bound to the gold nanoparticles with an amine group present on the surface of a bacteriophage, wherein the linker is (2S)-1-[(2S)-2-methyl-3-sulfanylpropanoyl]pyrrolidine-2-carboxylic acid (captopril).

In the present invention, step (a) may be performed by treating the linker with N-hydroxysuccinimide (NHS) and N-ethyl-N'-(3-dimethylaminopropyl)carbodiimide (EDC).

In the present invention, the bacteriophage may be bacteriophage MS2, but is not limited thereto.

In the present invention, the linker is (2S)-1-[(2S)-2-methyl-3-sulfanylpropanoyl]pyrrolidine-2-carboxylic acid (captopril), but is not limited thereto.

In the present invention, the size of the gold nanoparticles may be 1 to 100 nm, but is not limited thereto.

In still another aspect, the present invention is directed to the use of the bacteriophage-based gold nanohalo structure.

That is, the present invention is directed to a SERS composition for detection of a target substance, the SERS composition comprising the gold nanohalo structure.

In the present invention, the target substance may be selected from the group consisting of natural compounds, synthetic compounds, DNA, RNA, peptides, enzymes, ligands and proteins, but is not limited thereto.

Since the gold nanohalo structure fabricated in the present invention may form hot spots at regular intervals, it may generate a reproducible SERS signal, and the efficiency thereof is also excellent. For example, a secondary antibody is immobilized on the SERS substrate of the present invention, and an antibody that binds to a specific antigen is bound to serum, and then allowed to react with the SERS substrate having the secondary antibody immobilized thereon. In this case, the specific antigen may be detected with high accuracy and sensitivity.

Alternatively, an antibody for detecting a specific antigen may be immobilized directly on the gold nanohalo structure of the present invention and added to serum, and then a SERS signal may be directly measured using a SERS dye.

In this case, the SERS dye is preferably ATP, R6G, or TAMRA Raman dye, but is not limited thereto.

The specific antigen as described above may be, for example, a biomarker for sepsis. The biomarker may be selected from procalcitonin (PCT), a C-reactive protein (CRP), and a soluble triggering receptor expressed on myeloid cells-1 (sTREM-1).

Sepsis can be caused not only by pathogenic infection but also by inflammatory responses due to infection (Reinhart et al., 25:609-634, 2012; Rittirsch et al., 8:776-787, 2008; Rascher et al., 59:251-258, 2014). In particular, since sepsis can cause acute complex organ failure, sepsis needs to be accurately diagnosed early for immediate treatment.

In the process of direct detection of pathogens, the blood culture method has disadvantages in that it takes a lot of time and has a low sensitivity of only 30%, and in that about 30% of the infected bacteria cannot be cultured (Calandra et al., 22:1538-1548, 2005). The detection of pathogens in the bloodstream using a multiplex real-time PCR technique has the risk of producing a false negative result for patients in whom bacterial DNA does not exist in the blood, and thus detection of sepsis-specific biomarker molecules has been considered.

However, a specific and sensitive detection method using only one type of specific biomarker has not yet been established. An alternative to this method is to use a group of biomarkers that are clinically meaningful (Gibot et al.,186:65-71, 2008). The three biomarkers, C-reactive protein, procalcitonin, and sTREM-1, have been approved as biomarkers of sepsis (Zachariah et al., 36:1507-1512, 2008).

Disclosed but not explicitly claimed is a SERS method of detecting a target substance using the gold nanohalo structure.

The present invention is also directed to the use of the gold nanohalo structure for SERS-based detection of a target substance.

Disclosed but not explicitly claimed is the use of the composition comprising the gold nanohalo structure for SERS-based detection of a target substance.

## Examples

Hereinafter, the present invention will be described in more detail with reference to examples.

### <Experimental Method>

### 1. Compounds, Media and Strains

LB-Miller media, N-hydroxysuccinimide (NHS), N-ethyl-N'-(3-dimethylaminopropyl)carbodiimide (EDC), polyethylene glycol-8000 (PEG-8000), sodium chloride (NaCl), dimethyl sulfoxide (DMSO), captopril, and phosphotungstic acid (PTA) were purchased from Sigma-Aldrich. Sucrose was purchased from Amresco. PBS buffer was purchased from Lonza. 5-nm gold nanospheres were purchased from BBI Solutions.

*E. coli* (ATCC-15597) and bacteriophage MS2 (ATCC-15597-B1) were obtained from the ATCC.

### Example 1. Culture of Bacteriophage MS2

MS2 phage was cultured in *E. coli* according to the existing literature (J.M. Hooker et al., 2004, Journal of American Chemical Society, 126, 3718-3719). After 24 hours of culture, the *E. coli*-phage suspension was centrifuged at 5,000 ×g at 4°C for 30 minutes, and the supernatant containing the MS2 phage was isolated. PEG-8000 and NaCl were added to the isolated MS2 suspension to concentrations of 9% and 5.8% (w/v), respectively. The solution was stored at 4°C for about 16 hours so that the MS2 phage precipitated. The precipitated MS2 phage was isolated by centrifugation at 10,000 ×g at 4°C for 1 hour. The isolated MS2 phage was filtered using a centrifugal filter (Spin-X UF Concentrators 100K MWCO, Corning), and stored in a PBS buffer solution.

The concentration of the MS2 phage was measured based on the absorbance at a wavelength of 260 nm using a UV-Visible spectrophotometer (UV-3600, Shimadzu), and stored and used at a concentration of about 10 pfu/mL in the experiment.

### Example 2. Fabrication of Gold Nanohalo Structure

Captopril is a molecule having has two types of functional groups (a carboxyl group and a thiol group), and is used to treat hypertension. In the present invention, captopril was used as a linker connecting between gold nanoparticles with MS2 phage for future medical applications. Captopril was prepared in DMSO at a concentration of 60 mM. The captopril stock solution was stored at -20°C.

To connect the thiol group of captopril to an amine group present on MS2 phage, captopril was treated with NHS-EDC. NHS and EDC were each dissolved in DMSO at a concentration of 120 mM, and the solution was mixed with the captopril stock solution at a volume ratio of 1:1 and allowed to react at room temperature for 30 minutes. 10 $\mu$L of the resulting solution was added to and reacted with 1.6 mL of a solution of 5-nm gold nanoparticles for 30 minutes, thereby binding the thiol group of captopril to the gold nanoparticles.

The gold nanoparticles surface-modified with captopril were centrifuged at 12,000 ×g at 4°C for 5 minutes to remove unreacted captopril, NHS and EDC. The gold nanoparticles were dispersed again in 1.6 mL of distilled water, mixed with 1 mL of the MS2 phage suspension, and allowed to react at room temperature for 24 hours, thereby fabricating a nanohalo structure.

The fabricated nanohalo structure was isolated by density-gradient centrifugation. 30%, 40% and 50% aqueous solutions of sucrose were prepared, and 2 mL of each solution was added to a 15-mL Falcon tube to form layers with different densities. 2 mL of a solution of the fabricated nanohalo structure was added thereto and centrifuged at 5,000 ×g at 4°C for 15 minutes to isolate the nanohalo structure from unreacted gold nanoparticles. The isolated nanohalo structure was washed three times with distilled water.

### Example 3. Analysis of Gold Nanohalo Structure

The 5-nm gold nanoparticles, the MS2 phage and the nanohalo structure were analyzed by dynamic light scattering, UV-Vis spectrophotometry, FT-IR and TEM. Dynamic light scattering, a method for measuring the size distribution of nano-sized particles, was performed using a Zetasizer Nano ZS90 (Malvern Panalytical). The spectra of the 5-nm gold nanoparticles and the nanohalo structure in the wavelength range of 400 to 800 nm were recorded using a UV-Vis spectrophotometer (UV-3600, Shimadzu).

FT-IR was used to confirm the formation of bonds between the MS2 phage, the captopril linker and the gold nanoparticles, and was recorded in the range of 650 to 4,000 cm$^{-1}$ using a Cary 630 FTIR Spectrometer (Agilent Technologies).

TEM images of the MS2 phage, the captopril linker and the nanohalo structure were obtained using a TECNAI 20 electron microscope (FEI). To obtain a TEM image of the MS2 phage, negative staining was performed. 5 $\mu$L of the MS2 phage suspension was dropped onto a copper-coated TEM grid (Ted Pella), and after 1 minute, the TEM grid was washed with distilled water. Thereafter, 5 $\mu$L of 1% PTA aqueous solution was dropped onto the TEM grid, and the MS2 phage was stained with PTA for 1 minute. Then, the TEM grid was washed again with distilled water and wiped with filter paper.

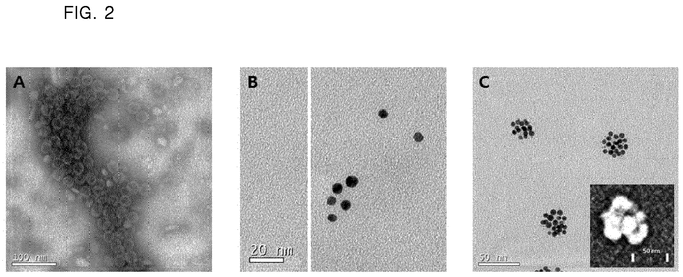

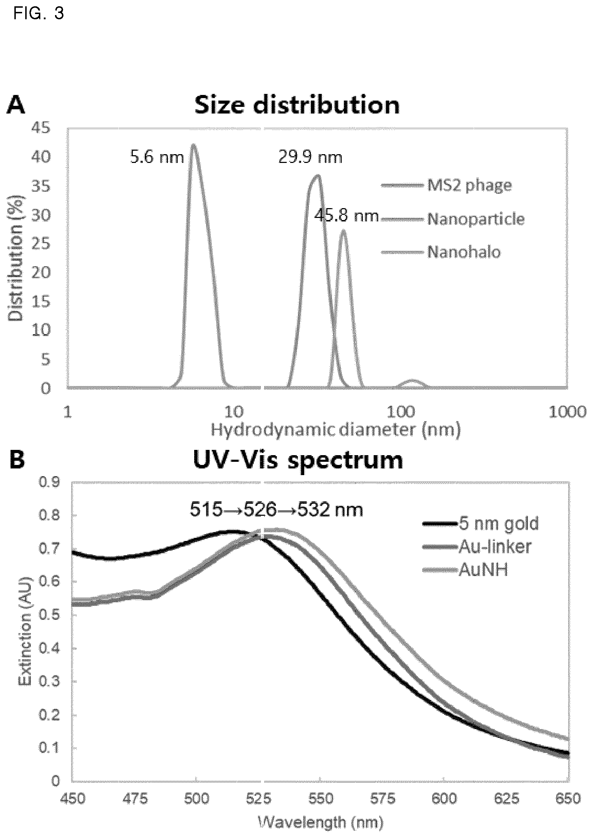

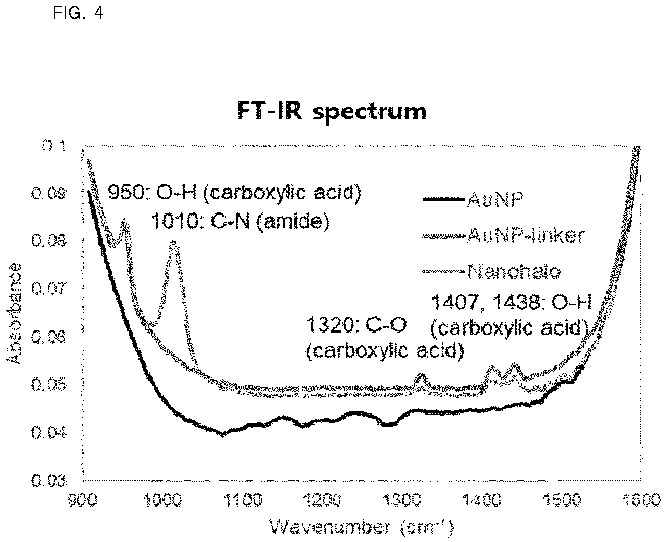

As a result, as shown in FIGS. 2 and 3, the gold nanoparticles were uniformly arranged on the surface of the bacteriophage, indicating that nanohalo structures having a uniform size and shape were formed. In addition, as shown in FIG. 4, it could be confirmed that the formation of these structures was due to the captopril linker.

### Example 4. Analysis of Raman Signal Amplification Effect of Gold Nanohalo Structure

The Raman signal amplification effect of the gold nanohalo structure was analyzed using an FEX spectroscopy system (NOST). The gold nanohalo structure was dropped onto a glass slide (Brain Research Laboratories) and dried. As an analyte for analysis of the Raman signal, malachite green, a type of Raman dye, was used. A 10$^{-2}$ M aqueous solution of malachite green oxalate (Sigma-Aldrich) was dropped onto the dried nanohalo structure on the glass slide and dried.

The dried sample was irradiated with a laser having a wavelength of 785 nm using a 100x objective lens (TU Plan ELWD 100x, Nikon, 0.6 NA, 0.56mm WD) of an upright microscope (Eclipse Ni-U, Nikon). The polarization direction of the laser was controlled by a linear polarizer (PRM 1/M, Thorlabs). The Raman spectrum generated from the analyte was measured using an FEX spectroscopy system and a CCD camera (Newton 920, Andor Technology).

As a control, the Raman spectrum of a sample obtained by dropping a $10^{-2}$ M aqueous solution of malachite green dye onto a glass substrate without the nanohalo structure was measured. The signal intensities of the two spectra were compared with each other to confirm the signal enhancement effect of the nanohalo structure. The enhancement factor, which is a measure of the SERS signal enhancement effect, was calculated using the following Equation 1.

$$\text{Equation } 1 = EF = \frac{I_{SERS}}{M_{SERS}/A_{SERS}} \times \frac{M_{Raman}/A_{Raman}}{I_{Raman}}$$

wherein I denotes the intensity of a specific peak in the spectrum, M denotes the concentration of the analyte, A denotes the area occupied by the analyte after drying, the subscript SERS denotes the spectrum with enhanced signal intensity, and the subscript Raman denotes the spectrum without signal enhancement.

As a result, as shown in FIG. 5, it can be confirmed that the gold nanohalo structure greatly amplified the Raman signal of the analyte. In addition, when the enhancement factor for the peak at 1178 $cm^{-1}$ is calculated, it can be confirmed that an arithmetically calculated signal amplification effect of $4.1 \times 10^7$ times appeared.

In addition, in order to confirm the reproducibility of the signal enhancement effect of the nanohalo structure, fabrication of the nanohalo structure was repeated three times through the same process, and the Raman spectra of the fabricated nanohalo structures were measured in the same manner. The intensities of the main peaks in the Raman spectrum of the analyte malachite green were compared.

As a result, as shown in FIG. 6, it can be confirmed that the SERS signal intensity due to the gold nanohalo structure was almost the same in all three measurements. In particular, it can be seen that the signal intensity consistently appeared within a standard deviation of 7% at the five peaks (1177, 1298, 1372, 1397 and 1593 $cm^{-1}$) selected as representatives.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims.

## Industrial Applicability

The gold nanohalo structure according to the present invention may generate a consistent SERS signal due to regular hot spots generated on the structure, and may be effectively used in the development of a SERS-based diagnostic kit.

## Claims

1. A gold nanohalo structure in which gold nanoparticles are bound via a linker containing a carboxyl group and a thiol group to a bacteriophage having an amino functional group arranged on a surface thereof,
   wherein the linker is (2S)-1-[(2S)-2-methyl-3-sulfanylpropanoyl]pyrrolidine-2-carboxylic acid (captopril).

2. The gold nanohalo structure of claim 1, wherein the gold nanohalo structure has surface-enhanced Raman scattering (SERS) activity.

3. The gold nanohalo structure of claim 1, wherein the bacteriophage is bacteriophage MS2.

4. The gold nanohalo structure of claim 1, wherein the gold nanoparticles have a size of 1 to 100 nm.

5. A method for fabricating a gold nanohalo structure, the method comprising steps of:

   (a) activating a carboxyl group of a linker containing the carboxyl group and a thiol group;
   (b) binding the thiol group of the linker to gold nanoparticles; and
   (c) reacting the carboxyl group of the linker bound to the gold nanoparticles with an amine group present on a surface of a bacteriophage,

wherein the linker is (2S)-1-[(2S)-2-methyl-3-sulfanylpropanoyl]pyrrolidine-2-carboxylic acid (captopril).

6. The method of claim 5, wherein step (a) is performed by treating the linker with N-hydroxysuccinimide (NHS) and N-ethyl-N'-(3-dimethylaminopropyl)carbodiimide (EDC).

7. The method of claim 5, wherein the bacteriophage is bacteriophage MS2.

8. The method of claim 5, wherein the gold nanoparticles have a size of 1 to 100 nm.

9. A SERS composition for detection of a target substance, the SERS composition comprising the gold nanohalo structure of claim 1.


**Patentansprüche**

1. Gold-Nanohalo-Struktur, in der Gold-Nanopartikel über einen Linker, der eine Carboxygruppe und eine Thiolgruppe enthält, an einen Bakteriophagen gebunden sind, der eine funktionelle Aminogruppe auf einer Oberfläche davon aufweist,
   wobei der Linker (2S)-1-[(2S)-2-Methyl-3-sulfanylpropanoyl]pyrrolidin-2-carbonsäure (Captopril) ist.

2. Gold-Nanohalo-Struktur nach Anspruch 1, wobei die Gold-Nanohalo-Struktur eine oberflächenverbesserte Raman-Streuungs- (SERS-) Aktivität aufweist.

3. Gold-Nanohalo-Struktur nach Anspruch 1, wobei der Bakteriophage der Bakteriophage MS2 ist.

4. Gold-Nanohalo-Struktur nach Anspruch 1, wobei die Gold-Nanopartikel eine Größe von 1 bis 100 nm aufweisen.

5. Verfahren zur Herstellung einer Gold-Nanohalo-Struktur, wobei das Verfahren die folgenden Schritte umfasst:

   (a) das Aktivieren einer Carboxylgruppe eines Linkers, der die Carboxylgruppe und eine Thiolgruppe enthält;
   (b) das Binden der Thiolgruppe des Linkers an Gold-Nanopartikel; und
   (c) das Umsetzen der Carboxylgruppe des Linkers, der an die Gold-Nanopartikel gebunden ist, mit einer Aminogruppe, die auf einer Oberfläche eines Bakteriophagen vorhanden ist,

   wobei der Linker (2S)-1-[(2S)-2-Methyl-3-sulfanylpropanoyl]pyrrolidin-2-carbonsäure (Captopril) ist.

6. Verfahren nach Anspruch 5, wobei Schritt (a) durch Versetzen des Linkers mit N-Hydroxysuccinimid (NHS) und N-Ethyl-N'-(3-dimethylaminopropyl)carbodiimid (EDC) durchgeführt wird.

7. Verfahren nach Anspruch 5, wobei der Bakteriophage der Bakteriophage MS2 ist.

8. Verfahren nach Anspruch 5, wobei die Gold-Nanopartikel eine Größe von 1 bis 100 nm aufweisen.

9. SERS-Zusammensetzung zur Detektion einer Zielsubstanz, wobei die SERS-Zusammensetzung eine Gold-Nanohalo-Struktur nach Anspruch 1 umfasst.


**Revendications**

1. Structure de nanohalo d'or dans laquelle des nanoparticules d'or sont liées via un lieur contenant un groupe carboxyle et un groupe thiol à un bactériophage présentant un groupe fonctionnel amino agencé sur une surface de celui-ci, dans laquelle le lieur est l'acide (2S)-1-[(2S)-2-méthyl-3-sulfanylpropanoyl]pyrrolidine-2-carboxylique (captopril).

2. Structure de nanohalo d'or selon la revendication 1, dans laquelle la structure de nanohalo d'or présente une activité de diffusion Raman exaltée par effet de surface (SERS).

**3.** Structure de nanohalo d'or selon la revendication 1, dans laquelle le bactériophage est le bactériophage MS2.

**4.** Structure de nanohalo d'or selon la revendication 1, dans lequel les nanoparticules d'or sont dans la plage de 1 à 100 nm.

**5.** Procédé de fabrication d'une structure de nanohalo d'or, le procédé comprenant les étapes consistant à :

(a) activer un groupe carboxyle d'un lieur contenant le groupe carboxyle et un groupe thiol ;
(b) lier le groupe thiol du lieur à des nanoparticules d'or ; et
(c) faire réagir le groupe carboxyle du lieur lié aux nanoparticules d'or avec un groupe amine présent sur une surface d'un bactériophage,

dans lequel le lieur est l'acide (2S)-1-[(2S)-2-méthyl-3-sulfanylpropanoyl]pyrrolidine-2-carboxylique (captopril).

**6.** Procédé selon la revendication 5, dans lequel l'étape (a) est effectuée en traitant le lieur avec du N-hydroxysuccinimide (NHS) et du N-éthyl-N'-(3-diméthylaminopropyl) carbodiimide (EDC).

**7.** Procédé selon la revendication 5, dans lequel le bactériophage est le bactériophage MS2.

**8.** Procédé selon la revendication 5, dans lequel les nanoparticules d'or présentent une taille de 1 à 100 nm.

**9.** Composition SERS pour la détection d'une substance cible, la composition de SERS comprenant la structure de nanohalo d'or selon la revendication 1.

FIG. 1

FIG. 2

FIG. 3

**A** Size distribution

**B** UV-Vis spectrum

FIG. 4

FT-IR spectrum

FIG. 5

**Raman spectrum**

FIG. 6

A — Raman spectrum

B — Raman peaks intensity

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011130332 A1

### Non-patent literature cited in the description

- **A.H. NGUYEN et al.** *Journal of Optics*, 2015, vol. 17, 114022
- **B. SHARMA et al.** *MRS Bulletin*, 2013, vol. 38, 615-624
- **H. LIU et al.** *Scientific Reports*, 2011, vol. 1, 112
- **S.L. CAPEHART et al.** *Journal of American Chemical Society*, 2013, vol. 135, 3011-3016
- **PARENT K N et al.** *Structure*, 10 March 2010, vol. 18 (3), 390-401
- **EVERTS MAAIKE et al.** Reveal a Novel Mechanism for Capsid Maturation. *Nano Lett.*, April 2006, vol. 6 (4), 587-91
- **J.M. HOOKER et al.** *Journal of American Chemical Society*, 2004, vol. 126, 3718-3719